# EUROPÄISCHE PATENTSCHRIFT

(11) **EP 1 200 137 B1**
(45) Veröffentlichungstag und Bekanntmachung des Hinweises auf die Patenterteilung: **19.03.2008**
(21) Anmeldenummer: 00953004.9
(22) Anmeldetag: 05.07.2000
(51) Int. Cl.: A61L 24/10, A61B 17/00

(54) **MITTEL ZUM VERSCHLUSS VON BLUTGEFÄSSEN**
AGENT FOR OCCLUDING BLOOD VESSELS
AGENT POUR L'OBTURATION DE VAISSEAUX SANGUINS

(30) Priorität: 06.07.1999 DE 29911689 U
(43) Veröffentlichungstag der Anmeldung: 02.05.2002
(73) Patentinhaber: Sterk, Axel, 88239 Wangen (DE)
(72) Erfinder: Sterk, Axel, 88239 Wangen (DE)
(74) Vertreter: Riebling, Peter
(86) Internationale Anmeldenummer: PCT/EP2000/006282
(87) Internationale Veröffentlichungsnummer: WO 2001/002029

(56) Entgegenhaltungen:
- WO-A-87/00062
- DE-A- 19 647 280
- DE-A- 19 731 741
- DE-A- 19 853 033
- US-A- 4 999 188
- US-A- 5 219 328
- US-A- 5 583 114
- US-A- 5 648 100
- DATABASE WPI Section Ch, Week 199338 Derwent Publications Ltd., London, GB; Class B04, AN 1993-297783 XP002152041 & JP 05 208917 A (TERUMO CORP), 20. August 1993 (1993-08-20)

## Beschreibung

Gegenstand der Erfindung ist ein Mittel zum Verschluss von Blutgefäßen, das den Erfolg von operativen Eingriffen, insbesondere den von operativen Eingriffen zur Entfernung von Karzinomen, erheblich verbessert.

Aus der europäischen Patentanmeldung 0 797 988 ist bereits ein Verfahren zur Embolisierung von Blutgefäßen bekannt, bei dem zur Behandlung eines Karzinoms eine antiangiogene Zubereitung in ein Blutgefäß, das den Tumor versorgt, eingebracht wird. Durch die "Embolisation" des Blutgefäßes wird die Blutzufuhr von dem erkrankten Gewebe in das gesunde Gewebe und umgekehrt unterbrochen.

Es ist außerdem in der DE-OS 197 31 741 bereits vorgeschlagen worden, bestimmte Konjugate, die eine fluoreszenzfähige Verbindung und einen Träger umfassen, zur Unterscheidung von gesundem und erkranktem Gewebe einzusetzen. Diese Druckschrift beschreibt ein Konjugat, umfassend eine zur Fluoreszenz fähige Verbindung und einen Träger, wobei die Verbindung und der Träger über eine Säureester-, eine Säureamid-Bindung oder eine Enan-Brücke verbunden sind. Der Träger umfasst Verbindungen jeglicher Art, die zur Anreicherung des Konjugats in einem bestimmten Gewebe, z. B. Tumor, einem Entzündungsherd oder in oberflächlich gelegenen kleineren Gefäßen, wie Neovaskularisationen im Bereich der Cornea, geeignet sind. Beispiele solcher Träger sind Proteine und Polyether. Die Konjugate reichern sich in krankhaftem Gewebe an. Durch Licht wird die zur Fluoreszenz fähige Verbindung angeregt, wodurch krankhaftes Gewebe sichtbar gemacht werden kann, wohingegen gesundes Gewebe, in dem sich die Konjugate nicht anreichern, nicht sichtbar gemacht wird. Ein Verschluss von Blutgefäßen wird in dieser Druckschrift nicht angesprochen. Weiterhin ist Gegenstand dieser Druckschrift lediglich ein Diagnosemittel zur Unterscheidung von erkranktem und gesundem Gewebe, da der Träger so ausgewählt wird, dass sich das Konjugat besonders im krankhaften Gewebe anreichert.

Die US-A-5,583,114 beschreibt zwar den Einsatz von Fibrinklebern, um Gewebe wie Blutgefäße zu versiegeln, ohne jedoch irgendeine Anregung zu geben, gleichzeitig einen Farbstoff anzuwenden, um das Gewebe sichtbar zu machen.

Die US-A-5,648,100 beschreibt ein Mittel zur therapeutischen Embolisierung von Blutgefäßen, das Mikrokügelchen aus einem hydrophilen Acrylatcopolymer enthält. Zur Sichtbarmachung der Mikrokügelchen enthält das Copolymer ein funktionalisiertes Monomer, das bei der Herstellung der Mikrokügelchen durch eine chemische Kupplung mit einem Farbstoff erhalten wird. Die Mikrokügelchen können auch nach ihrer Herstellung mit Fluoreszenzfarbstoffen, wie Erythrosin oder Fluoreszein, markiert werden. Diese Druckschrift lässt jeden Hinweis auf einen Fibrinkleber vermissen. Darüber hinaus wird dort der Farbstoff chemisch an die Mikrokügelchen gebunden, so dass die Substanz, die zur Embolisierung der Blutgefäße verwendet wird, selbst sichtbar ist. Damit werden die Nachteile von Substanzen, die nicht markiert sind, nämlich dass sie vor, während und nach ihrer Injektion in den Blutgefäßen für den Benutzer schwer sichtbar sind, gelöst. Diese Druckschrift spricht jedoch eine Abgrenzung zwischen den Geweben sichtbar zu machen, nicht an.

Auch Fibrinkleber haben sich schon als Mittel für den Gefäßverschluss bewährt. Bei dem Einsatz eines üblichen Fibrinklebers in der onkologischen Chirurgie ist es jedoch von Nachteil, dass dabei die Unterscheidung zwischen dem zu entfernenden kranken Gewebe und dem gesunden Gewebe bisher sehr schwierig oder unmöglich sein kann.

Ausgehend von der Druckschrift US-A-5,648,100 als nächstliegendem Stand der Technik liegt der Erfindung die Aufgabe zugrunde, ein weiteres Mittel zum Verschluss von Blutgefäßen mit gleichzeitiger sichtbarer Abgrenzung des durch Unterbrechung der Blutzufuhr nicht versorgten Gewebes zur Verfügung zu stellen.

Zur Lösung dieser Aufgabe schlägt die Erfindung das Mittel gemäss Anspruch 1 vor, welches durch den Einsatz des dort näher definierten Fibrinklebers und eines Farbstoffes in Lösung gekennzeichnet ist.

Das erfindungsgemäße Mittel zum Verschluss von Blutgefäßen enthält ein Mittel zur Herbeiführung eines Gefäßverschlusses und einen physiologisch verträglichen Farbstoff. Das Mittel, das zur Herbeiführung des Gefäßverschlusses dient, enthält eine flüssige Fibrinogen-Zubereitung, die zusammen mit einer flüssigen Thrombin-Zubereitung angewendet wird.

Der physiologisch verträgliche Farbstoff wird zweckmäßigerweise einer der beiden Zubereitungen, im allgemeinen der Thrombin-Zubereitung, zugesetzt.

Bei der Anwendung des erfindungsgemäßen Mittels ergibt sich der Vorteil, dass es damit möglich wird, die einzelnen Blutbahnen nicht nur zu verschließen, sondern auch anzufärben und damit die Blut- oder lymphatische Versorgung sichtbar darzustellen. Es kann sowohl venöse als auch arterielle Blutgefäße verschließen und anfärben, kann aber auch in lymphatischen Gefäßen eingesetzt werden.

Mit dem erfindungsgemäßen Mittel ist es außerdem möglich, gesundes von krankem Gewebe sichtbar voneinander abzugrenzen. Da jedes Gewebe von einer bestimmten Arterie und Vene und von einer bestimmten Lymphbahn versorgt wird, kann es von der Blutzufuhr abgeschnitten werden, wenn die entsprechende zuführende oder abführende Versorgungsbahn embolisiert wird. Hierbei ist es gleichgültig, welche Gewebebahn embolisiert wird. Wichtig ist nur, dass die Blutzufuhr zu dem erkrankten

Gewebe unterbrochen wird, was sowohl durch die Embolisation der arteriellen als auch der venösen Gewebebahnen erreicht werden kann. Die Verwendung des erfindungsgemäßen Mittels führt damit bei operativen Eingriffen zum Verschluss der das Operationsfeld versorgenden Gefäße.

Für den Chirurgen wird durch die Anwendung des erfindungsgemäßen Mittels der Eingriff erheblich erleichtert, weil er nun während der Operation das kranke Gewebe vom gesunden Gewebe ohne weiteres unterscheiden und bei Abtrennung des kranken Gewebes das gesunde Gewebe weitestgehend erhalten kann.

Ein weiterer Vorteil des angegebenen Mittels besteht darin, das es durch den Verschluss der das Operationsfeld versorgenden Blutgefäße eine Diffusion von pathogenen Keimen oder Körperzellen in das gesunde Körpergewebe verhindert. Insbesondere Bakterien, Viren und Tumorzellen werden somit in dem kranken Gewebe fixiert. Die gleichen Vorteile ergeben sich bei einem durch Parasitenbefall erkranktem Gewebe, weil auch in diesem Fall die Verbindung des gesunden Gewebes mit dem kranken Gewebe unterbrochen wird.

Der erfindungsgemäß einsetzbare Gewebekleber besteht vorzugsweise aus einer stabilisierten, flüssigen Fibrinogen- und einer flüssigen Thrombinzubereitung. Eine oder beide dieser Zubereitungen sollen einen physiologisch verträglichen Farbstoff enthalten, der die embolisierten Blutgefäße deutlich anfärbt. Geeignete Farbstoffe sind bspw. Methylenblau, Chinolongelb, Patentblau, Toloniumchlorid, Indocyaningrün und Lebensmittel- sowie Fluoreszenzfarbstoffe.

Dabei kann dem Gewebekleber zusätzlich eine den Blutgerinnungsfaktor XIII enthaltende Zubereitung zugesetzt sein, so dass er als 3-Komponenten-Kleber verwendet wird. Es ist allerdings auch möglich, den Blutgerinnungsfaktor XIII von Anfang an der Fibrinogenzubereitung zuzumischen, so dass ein 2-Komponenten-Kleber zum Einsatz kommt. Im Falle eines 3-Komponentenklebers kann das Mischungsverhältnis der Komponenten Fibrinogen, Faktor XIII und Thrombin in geeigneter Weise gewählt werden, um gute mechanische Eigenschaften des Klebers zu erzielen. Geeignet sind z.B. Mischungsverhältnisse von 1:1:1 und ca. 2:1:1 bis ca. 10:1:1.

Der erfindungsgemäß verwendete Gewebekleber enthält in der Fibrinogenzubereitung eine chaotrope Substanz. Als geeignete chaotrope Substanzen haben sich vor allem Arginin, Guanidin, Citrullin, Harnstoff oder dessen Derivate oder ihre Mischungen erwiesen. Sie werden der Fibrinogenzubereitung im allgemeinen in Mengen von 0,1 bis 1,0 Mol/l, vorzugsweise in Mengen von unter 0,5 Mol/l, beigegeben.

Die Eigenschaften der vorstehend genannten neuen Gewebekleber werden weiterhin durch den Zusatz eines Antifibrinolytikums vorteilhaft beeinflusst. Als Antifibrinolytikum werden vor allem Aprotinin, e-Aminocapronsäure (EACA), p-Aminomethylbenzoesäure (PAMBA) oder eines ihrer physiologisch verträglichen Salze oder Derivate verwendet.

Außerdem können in der Fibrinogenzubereitung als Stabilisatoren
- ein anorganisches Salz oder
- ein oder mehrere physiologisch verträgliche Salze von organischen Carbonsäuren, insbesondere der Zitronensäure oder der Milchsäure, oder
- eine oder mehrere Aminosäuren oder
- ein Mono- oder Disaccharid oder
- ein Zuckeralkohol
oder eine ihrer Mischungen enthalten sein.

Die dem erfindungsgemäß einzusetzenden Gewebekleber hinzugefügte Faktor XIII-Zubereitung muss ebenfalls stabilisiert werden, wenn sie nicht dem bereits stabilisierten Fibrinogen zugegeben wird. In diesem Fall ist es vorteilhaft, der Faktor XIII-Zubereitung ein physiologisch verträgliches Salz einer organischen Di-, Tri- oder Tetracarbonsäure, insbesondere der Zitronensäure, und gegebenenfalls weitere Stabilisatoren und/oder Puffersubstanzen für den Faktor XIII zuzugeben. Als weitere Stabilisatoren kommen dabei
- ein Mono- oder Disaccharid oder ein Zuckeralkohol und/oder
- eine Aminosäure aus der Gruppe Glyzin, Glycylglyzin, Alanin, Cystein, Histidin, Glutamin oder ein physiologisch verträgliches Salz der Glutamin- oder Asparaginsäure und/oder
- ein reduzierendes oder oxidationsverhinderndes Agens und/oder
- eine oberflächenaktive Substanz in Betracht.

Sie werden üblicherweise in einer Menge von bis zu 5 Gew.% der Faktor XIII-Zubereitung zugesetzt. Gewebekleber dieser Art sind in den deutschen Patentanmeldungen DE-A-198 53 033 und DE-A-198 61 158 beschrieben.

Die Erfindung wird durch die beigefügten Beispiele näher erläutert.

Es zeigen:
- Fig. 1: die Darstellung von zwei Stechflaschen mit unterschiedlichen Inhaltsstoffen,
- Fig. 2: die Stechflaschen nach Fig. 1 unter Hinzufügung weiterer Zusatzmittel,
- Fig. 3: die Anwendung des Mittels in einer ersten Ausführungsform,
- Fig. 4: die Anwendung des Mittels in einer zweiten Ausführungsform, und
- Fig. 5: die Erläuterung der Wirkungsweise des Mittels im Gewebe.

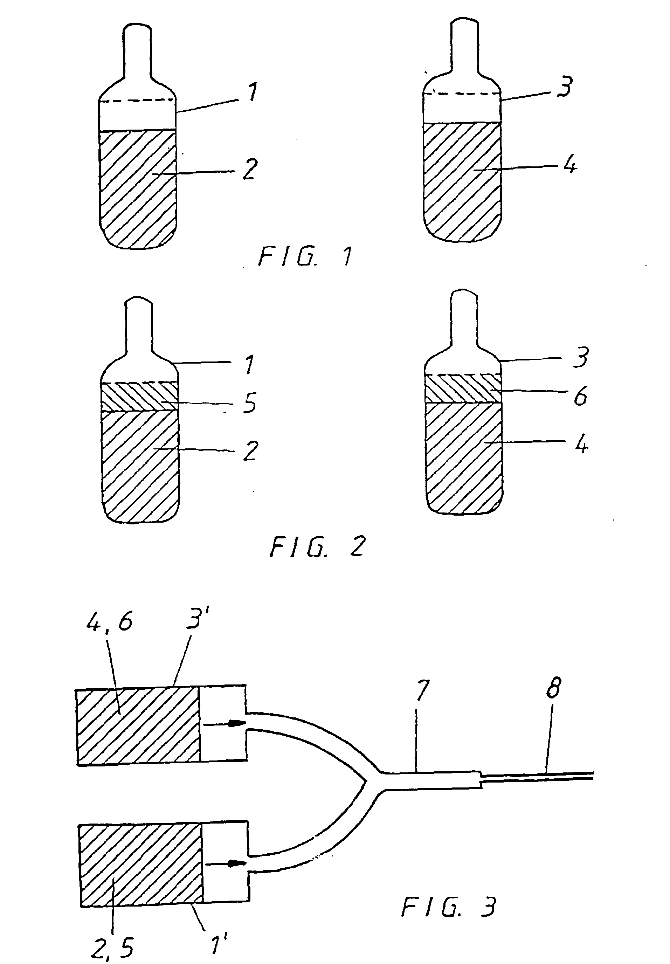

In Fig. 1 sind zwei Stechflaschen 1 und 3 dargestellt, die in unterschiedlicher Form gestaltet sein können. Die Stechflasche 1 kann eine Thrombin-Lösung 2 enthalten, in der ein physiologisch verträglicher Farbstoff in Verbindung mit Thrombin gelöst ist. Hierauf ist die Erfindung jedoch nicht beschränkt; die Stechflasche 1 kann auch lediglich eine Farbstofflösung enthalten. Die Beifügung von Thrombin ist nur zur Verbesserung der Blutgerinnung vorgesehen, ist aber nicht unbedingt notwendig für das erfindungsgemäße Mittel.

Die Stechflasche 3 enthält eine Lösung von Fibrinogen. Das Fibrinogen liegt in einer zähflüssigen, hochviskosen Lösung vor.

Zur Herstellung des erfindungsgemäßen Mittels wird nun in die Stechflasche 1 ein Zusatzmittel 5 eingefüllt, welches bevorzugt aus einer CaCl₂-Lösung besteht und zur späteren Aushärtung des Mittels im Gewebe dient.

In die zweite Stechflasche 3 wird als Zusatzmittel 6 eine Aprotinin-Lösung gegeben. Ein Mischungsverhältnis der Aprotinin-Lösung zur Fibrinogen-Lösung von 1:1 ist bevorzugt.

Das Zusatzmittel 6 (Aprotinin-Lösung) für das Fibrinogen ist erforderlich, um die später erwünschte Gerinnungskette in Gangzu setzen.

Die Inhaltsstoffe der Stechflaschen 1 und 3 reagieren zunächst noch nicht miteinander.

Erst wenn gemäß Fig. 3 der Inhalt der beiden Stechflaschen 1 und 3 in die ihnen zugeordneten Spritzen 1' und 3' aufgezogen wird und diese durch einen Y-Verbinder gemäß Fig. 3 verbunden werden, erfolgt eine Reaktion, sobald der Inhalt der beiden Spritzen 1' und 3' über den Y-Verbinder 7 und eine Kanüle 8 in das Gewebe eingespritzt wird.

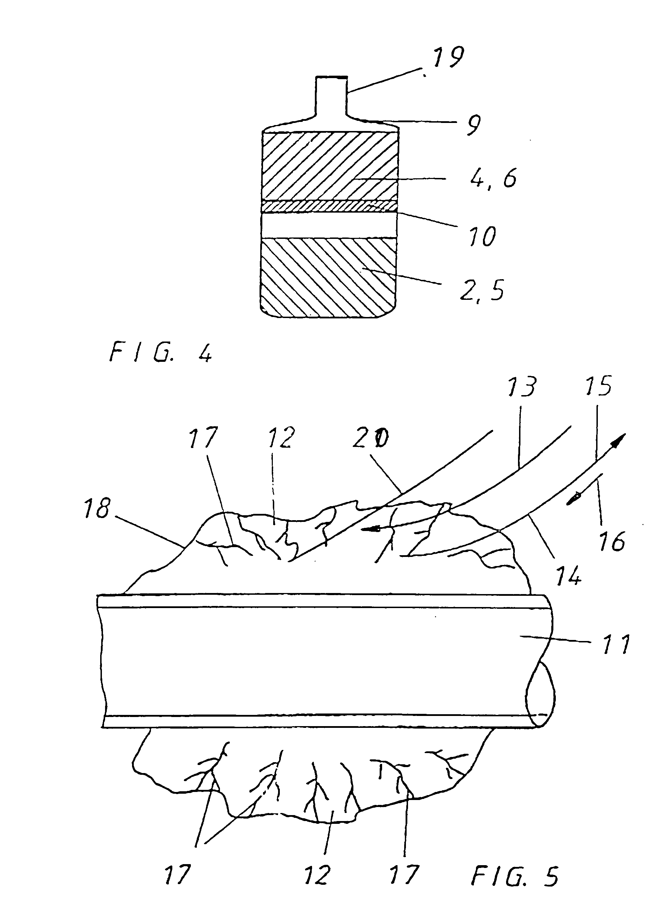

In Fig. 4 ist als weitere Ausführungsform ein Kombinationsgefäß 9 dargestellt, welches die Komponenten der beiden Stechflaschen 1 und 3 in der Ausführung nach Fig. 2 enthält.

Im oberen Teil kann es den Inhalt der Stechflasche 3 enthalten, während sich im unteren Teil des Kombinationsgefäßes 9 die Inhaltsstoffe der Stechflasche 1 befinden. Die beiden Komponenten sind durch eine mittlere Membran 10 voneinander getrennt.

Ein derartiges Kombinationsgefäß wird in der Form angewendet, dass die mittlere, trennende Membran 10 zerstört und das Kombinationsgefäß dann so geschüttelt wird, dass sich alle Komponenten miteinander vermischen. Danach kann das so hergestellte Mittel durch die Öffnung 19 und eine entsprechende Kanüle 8 in das Gewebe eingespritzt werden.

In dem Kombinationsgefäß 9 können statt einer horizontalen Membran auch mehrere horizontale Membranen oder auch eine oder mehrere vertikale Membranen vorhanden sein.

In Fig. 5 ist beispielhaft die Anwendung des Mittels an einem Enddarm 11 dargestellt. Die Anwendung des Mittels ist hierauf jedoch nicht beschränkt; es können sowohl lebende als auch tote Gewebe am Menschen und am Tierkörper mit dem erfindungsgemäßen Mittel behandelt werden.

Aus Fig. 5 ist erkennbar, dass bspw. bei Position 15, also weit außerhalb des kranken Geweben, in eine Vene 14 das Mittel aus der Kanüle 8 unter Druck eingespritzt wird, so dass es in Pfeilrichtung 16 entgegen der Blutflussrichtung in der Vene 14 fließt.

Dadurch werden alle venösen Bahnen (Venolen 17) im befallenen, kranken Gewebe 12 angefärbt und gleichzeitig verschlossen. Damit entsteht die Möglichkeit, das Gewebe 12 von dem umliegenden Gewebe, welches nicht von der Vene 14 versorgt wird, abzugrenzen. Das umliegende Gewebe ist also durch eine Gewebegrenze 18 von dem kranken Gewebe 12 getrennt und leicht unterscheidbar. So kann durch einfache optische Kontrolle während der Operation das kranke Gewebe 12 aus dem umliegenden, gesunden Gewebe entfernt werden.

Ein weiterer wesentlicher Vorteil der erfindungsgemäßen Mittels besteht darin, dass das kranke Gewebe wenigstens im Rand- oder Grenzbereich verschlossene Gefäße aufweist, in denen Krankheitserreger immobil fixiert sind und dadurch nicht in gesundes, noch nicht befallenes Gewebe eindringen können.

Das erfindungsgemäße Mittel kann aber auch in eine Arterie 13 eingespritzt werden und dann ebenfalls in Pfeilrichtung 16 in die arteriellen Bahnen des Gewebes 12 eindringen, wobei es dort die arteriellen Bahnen dauerhaft verschließt und gleichzeitig anfärbt.

Wichtig bei der vorliegenden Erfindung ist also, dass das Mittel mindestens aus zwei Komponenten besteht, nämlich aus einem Stoff, welcher geeignet ist, eine Embolisation des Gewebes herbeizuführen und ferner aus einem Farbstoff, welcher geeignet ist, das entsprechende verschlossene Gewebe während des Verschlusses auch anzufärben.

### Bezugszeichenliste:

- 1: Stechflasche
- 2: Füllung (Farbstofflösung mit oder ohne Thrombin)
- 3: Stechflasche
- 4: Füllung (Fibrinogen)
- 5: Zusatzmittel (CaCl₂)
- 6: Zusatzmittel (Aprotinin-Lösung)
- 7: Y-Verbinder
- 8: Kanüle
- 9: Kombinationsgefäß
- 10: Membran
- 11: Enddarm
- 12: Gewebe
- 13: Arterie
- 14: Vene
- 15: Position
- 16: Pfeilrichtung
- 17: Venolen
- 18: Gewebegrenze
- 19: Öffnung
- 20: Lymphbahn

## Patentansprüche

1. Mittel zum Verschluss von Blutgefäßen, **dadurch gekennzeichnet, dass** es besteht aus
- einem physiologisch verträglichen Farbstoff in Lösung,
- einer stabilisierten, flüssigen Fibrinogen- und einer flüssigen Thrombinzubereitung, wobei die Fibrinogenzubereitung eine chaotrope Substanz enthält,
- einer CaCl₂-Lösung, die zur späteren Aushärtung des Mittels im Gewebe dient,
- einer Aprotinin-Lösung und gegebenenfalls zusätzlich einer den Blutgerinnungsfaktor XIII enthaltenden Zubereitung.

2. Mittel nach Anspruch 1, **dadurch gekennzeichnet, dass** es als chaotrope Substanz Arginin, Guanidin, Citrullin, Harnstoff oder dessen Derivate oder ihre Mischungen enthält.

3. Mittel nach den Ansprüchen 1 und 2, **dadurch gekennzeichnet, dass** es einen Farbstoff ausgewählt aus der Gruppe Methylenblau, Chinolongelb, Patentblau, Toloniumchlorid, Indocyaningrün, sowie der Lebensmittel- oder Fluoreszenzfarbstoffe enthält.

## Claims

1. Agent for occluding blood vessels, **characterized in that** it consists of
- a physiologically tolerated dye in solution,
- a stabilized, liquid fibrinogen preparation and a liquid thrombin preparation, the fibrinogen preparation containing a chaotropic substance,
- a CaCl₂ solution which serves for subsequent hardening of the agent in the tissue,
- an aprotinin solution and optionally additionally a preparation containing the blood clotting factor XIII.

2. Agent according to Claim 1, **characterized in that** it contains arginine, guanidine, citrulline, urea or derivatives thereof or their mixtures as the chaotropic substance.

3. Agent according to Claims 1 and 2, **characterized in that** it contains a dye selected from the group consisting of methylene blue, quinolone yellow, patent blue, tolonium chloride, indocyanine green and the food or fluorescent dyes.

## Revendications

1. Agent pour obturer des vaisseaux sanguins, **caractérisé en ce qu'**il se compose
- d'un colorant physiologiquement compatible, en solution,
- d'une préparation liquide stabilisée de fibrinogène et d'une préparation liquide de thrombine, la préparation de fibrinogène contenant une substance chaotrope,
- d'une solution CaCl₂ qui sert au durcissement ultérieur de l'agent dans le tissu,
- d'une solution d'aprotinine et éventuellement, en supplément, d'une préparation contenant le facteur de coagulation sanguine XIII.

2. Agent selon la revendication 1, **caractérisé en ce qu'**il contient comme substance chaotrope de l'arginine, de la guanidine, de la citrulline, de l'urée ou ses dérivés, ou des mélanges de ceux-ci.

3. Agent selon les revendications 1 et 2, **caractérisé en ce qu'**il contient un colorant choisi dans le groupe constitué par le bleu de méthylène, le jaune de quinolone, le bleu patenté V, le chlorure de tolonium, le vert d'indocyanine ainsi que les colorants alimentaires ou les colorants fluorescents.
